# EUROPÄISCHE PATENTSCHRIFT

(11) **EP 2 549 979 B1**
(45) Veröffentlichungstag und Bekanntmachung des Hinweises auf die Patenterteilung: **22.05.2019**
(21) Anmeldenummer: 11704071.7
(22) Anmeldetag: 21.02.2011
(51) Int. Cl.: A61Q 19/02, A61K 8/49, C07D 277/48, C07D 417/12

(54) **KOSMETISCHE ODER DERMATOLOGISCHE ZUBEREITUNGEN MIT EINEM GEHALT AN EINEM ODER MEHREREN THIAZOLDERIVATEN**
COSMETIC OR DERMATOLOGICAL PREPARATIONS WITH A CONTENT OF ONE OR MORE THIAZOLE DERIVATIVES
PRÉPARATIONS COSMÉTIQUES OU DERMATOLOGIQUES CONTENANT UN OU PLUSIEURS DÉRIVÉS DE THIAZOL

(30) Priorität: 23.03.2010 DE 102010012594
(43) Veröffentlichungstag der Anmeldung: 30.01.2013
(73) Patentinhaber: Beiersdorf AG, 20253 Hamburg (DE)
(72) Erfinder: KOLBE, Ludger, 21255 Dohren (DE); SCHERNER, Cathrin, 22844 Norderstedt (DE)
(86) Internationale Anmeldenummer: PCT/EP2011/052479
(87) Internationale Veröffentlichungsnummer: WO 2011/117034

(56) Entgegenhaltungen:
- EP-A1- 1 649 852
- WO-A1-2004/014903
- US-A1- 2003 158 199
- GERMANAS ET AL: "Discovery of small-molecule inhibitors of tyrosinase", BIOORGANIC & MEDICINAL CHEMISTRY LETTERS, PERGAMON, ELSEVIER SCIENCE, GB, Bd. 17, Nr. 24, 12. Oktober 2007 (2007-10-12), Seiten 6871-6875, XP022339589, ISSN: 0960-894X, DOI: 10.1016/J.BMCL.2007.10.014
- LYNCH D E ET AL: "The hydrogen-bonding networks of 2-amino-4-phenyl-1,3-thiazole derivatives", CRYSTAL ENGINEERING, ELSEVIER SCIENCE PUBLISHERS, BARKING, GB, Bd. 5, Nr. 2, 1. Juni 2002 (2002-06-01), Seiten 123-136, XP004394108, ISSN: 1463-0184, DOI: 10.1016/S1463-0184(02)00011-4
- DATABASE REGISTRY; CHEMICAL ABSTRA [Online] 22. September 2010 (2010-09-22), CHEMICAL LIBRARY ET AL: XP002687444, gefunden im REGISTRY Database accession no. 1242267-72-6

## Beschreibung

Die vorliegende Erfindung betrifft kosmetische Kosmetische oder dermatologische Zubereitungen mit einem Gehalt an an einem oder mehreren Thiazolen gegen unerwünschte Pigmentierung der Haut.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephel*ides), genetische Disposition, Fehlpigmentierung der Haut bei der Wundheilung bzw. -vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte "Triformula" entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Ziel der nachfolgenden Erfindung war es also, dem nachteiligen Stand der Technik Abhilfe zu verschaffen.

Die Lösung der Aufgaben, der der Erfindung zugrunde liegen, besteht in kosmetischen oder dermatologischen Zubereitungen mit einem wirksamen Gehalt an einem oder mehreren Thiazolen, welches oder welche gewählt wird oder werden aus der Gruppe der folgenden Substanzen:

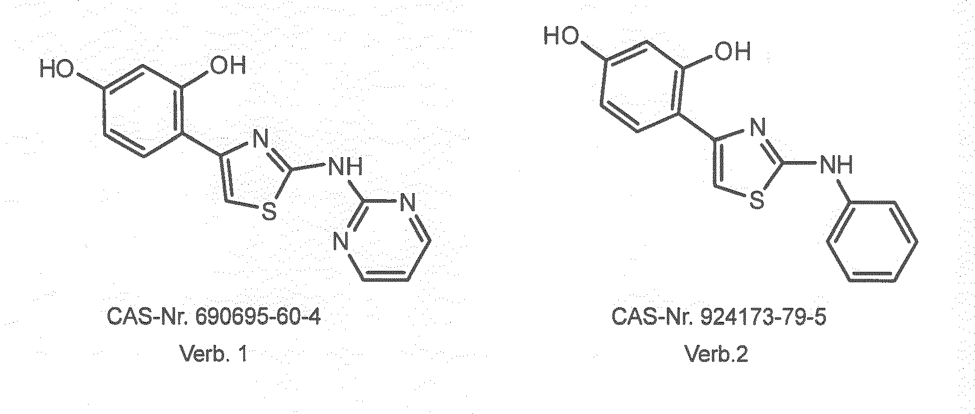

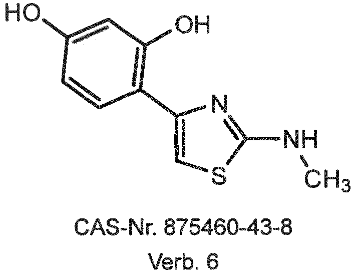

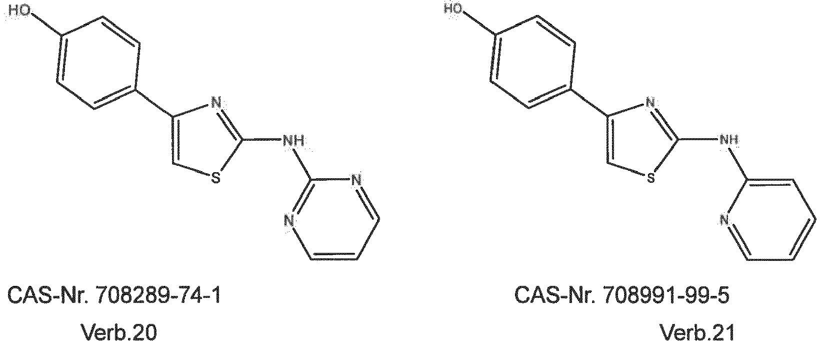

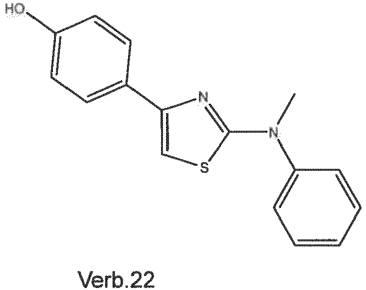

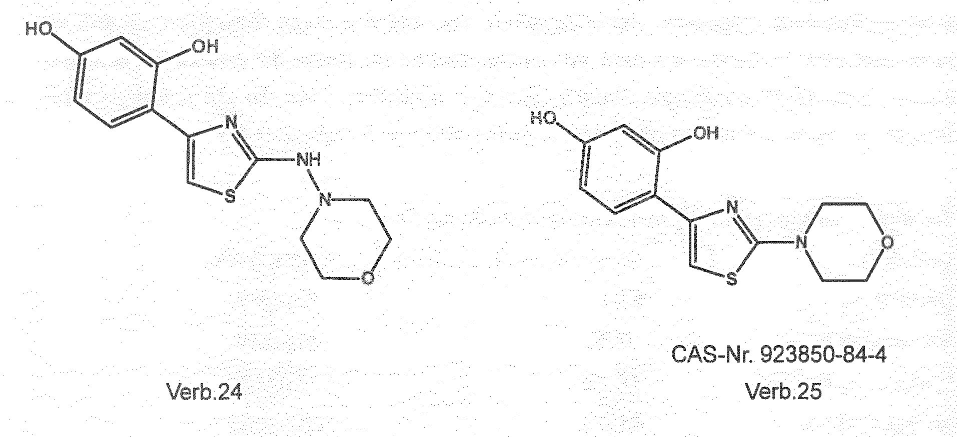

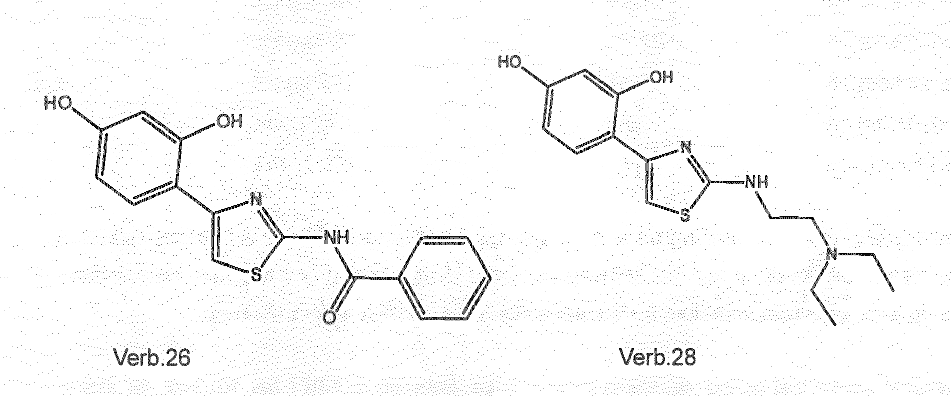

Die Arbeit "Discovery of small-molecule inhibitors of tyrosinase" in "Bioorganic & Medical Chemistry Letters 17 (2007) S. 6871 - 6875 konnte nicht den Weg zur vorliegenden Erfindung weisen

Die Synthese von Thiazolen ist in der chemischen Literatur sehr gut beschrieben, und die Darstellung der Substanzen deshalb für den geübten Chemiker problemlos möglich. Viele der von uns beschriebenen Substanzen sind mit einer CAS-Nr. eindeutig gekennzeichnet und können bei verschiedenen Herstellern von Feinchemikalien bezogen werden.

Die Wirksamkeit der Thiazole wurde mit einem Enzymtest belegt, in der Umsatz von L-DOPA zu L-Dopachinon durch eine humane Tyrosinase gemessen wurde. Bei dieser literaturbekannten Methode (Winder, A.J. and Harris, H., New assays for the tyrosine hydroxylase and dopa oxidase activities of tyrosinase. Eur. J. Biochem. (1991), 198, 317-26) wird das Reaktionsprodukt LDopaquinone mit MBTH (3methyl2benzothiazoline hydrazone) zu einer pinkfarbenen Substanz umgesetzt, deren Zunahme über die Zeit durch Absorption bei 490 nm gemessen wird. In Tabelle ein sind Wirksamkeitsdaten für einige der beanspruchten Substanzen beispielhaft dargestellt. Daraus lässt sich schließen, dass die erfindungsgemäßen Substanzen äußerst effektive pigmentierungs-inhibierende Substanzen sind.

| **Tabelle 1: Inhibition der Tyrosinaseaktivität durch Thiazole** | | |
|---|---|---|
| *Substanz* | *Inhibition (% der Kontrolle*) | *Konzentration* |
| Verbindung 1 | 93,1 | 100 µg/mL |
| Verbindung 2 | 100% | 100 µg/mL |
| Verbindung 6 | 87,4% | 100 µg/mL |
| Verbindung 20 | 61,3% | 100 µg/mL |
| Verbindung 21 | 87,6% | 100 µg/mL |
| Verbindung 22 | 87,9% | 100 µg/mL |
| Verbindung 24 | 82,9% | 100 µg/mL |
| Verbindung 25 | 90,3% | 100 µg/mL |
| Verbindung 26 | 100% | 100 µg/mL |
| Verbindung 28 | 80,5% | 100 µg/mL |

Kosmetische oder dermatologische Zubereitungen mit einem Gehalt an Thiazolderivaten bzw. deren Verwendung zur Behandlung und/oder Prophylaxe unerwünschter Hautpigmentierung, sind ebenfalls vorteilhafte Verkörperungen der vorliegenden Erfindung.

Vorteilhaft ist es insbesondere, wenn solche Zubereitungen 0,00001 bis 10 Gew.-%, insbesondere 0,001 bis 3 Gew.-%, ganz besonders 0,005 bis 1 Gew.-% an einem oder mehreren der erfindungsgemäß verwendeten Thiazolderivate enthalten, bezogen auf das Gesamtgewicht der Zubereitung.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/ W), eine multiple Emulsionen, beispielsweise vom Typ Wasser-in-ÖI-in-Wasser (W/O/W), ein Gel, einen festen Stift, eine Salbe oder auch ein Aerosol darstellen. Es ist auch erfindungsgemäß vorteilhaft, die erfindungsgemäß verwendeten Substanzen und/oder deren Derivate in verkapselter Form darzureichen, z.B. in Kollagenmatrices und anderen üblichen Verkapselungsmaterialien, z.B. als Celluloseverkapselungen, in Gelatine oder liposomal verkapselt. Es ist auch möglich und vorteilhaft im Sinne der vorliegenden Erfindung, die erfindungsgemäß verwendeten Substanzen und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethy-lhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Feuchthaltemittel wie z.B. Propylenglycol oder Panthenol sowie insbesondere ein oder mehrere Verdickungsmittel, welches oder welche vorteilhaft gewählt werden können aus der Gruppe Siliciumdioxid, Aluminiumsilikate, Hydroxypropylmethylcellulose, besonders vorteilhaft ein Polyacrylat wie beispielsweise Carbopole Typ 980, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Propyleglycol, und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

**O/W-Emulsion**

| | Anteil (%) |
|---|---|
| Cetearylglucosid | 1.5 |
| Cetearylalkohol | 5.0 |
| Caprylic/Capric Triglycerid | 3.0 |
| Dicaprylylcarbonat | 4.0 |
| C12-15 Alkylbenzoat | 3.0 |
| Cyclomethicon | 2.0 |
| Propylenglycol | 5.0 |
| Panthenol | 1.0 |
| Natrium Hydroxid | q.s. |
| 4-(2-(pyrimidin-2-ylamino)thiazol-4-yl)benzene-1,3-diol | 0.1 |
| Wasser | Ad 100 |

| | |
|---|---|
| pH-Wert: 5-6 | |

## Patentansprüche

1. Kosmetische oder dermatologische Zubereitungen mit einem wirksamen Gehalt an einem oder mehreren Thiazolen welches oder welche gewählt wird oder werden aus der Gruppe der folgenden Substanzen:

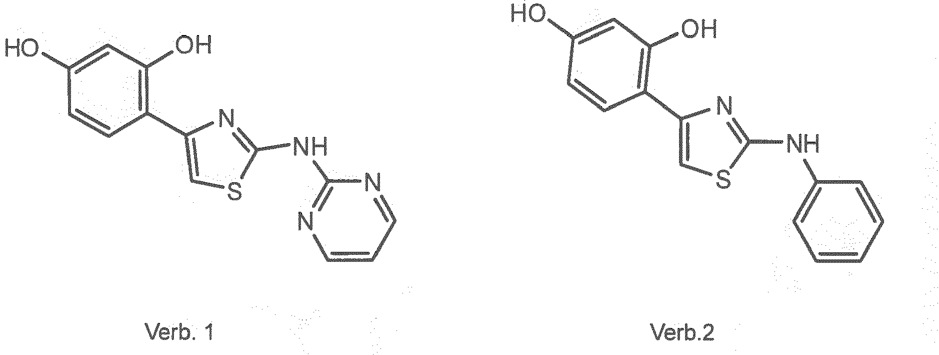

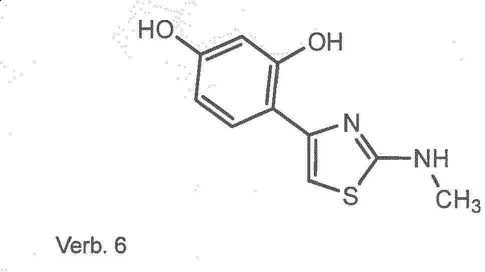

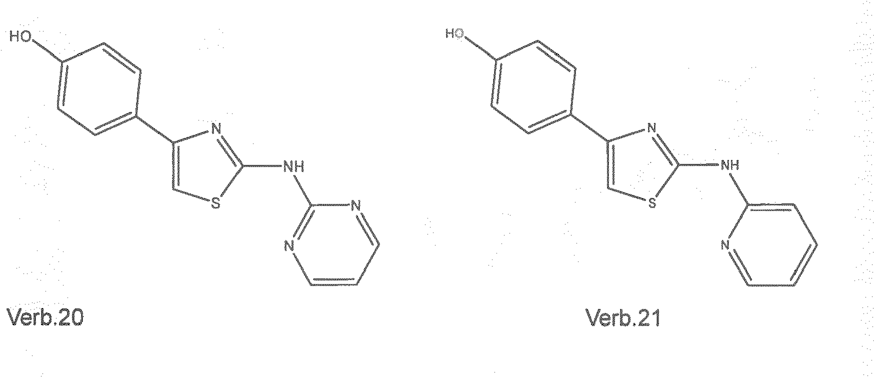

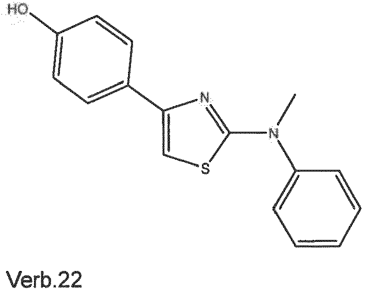

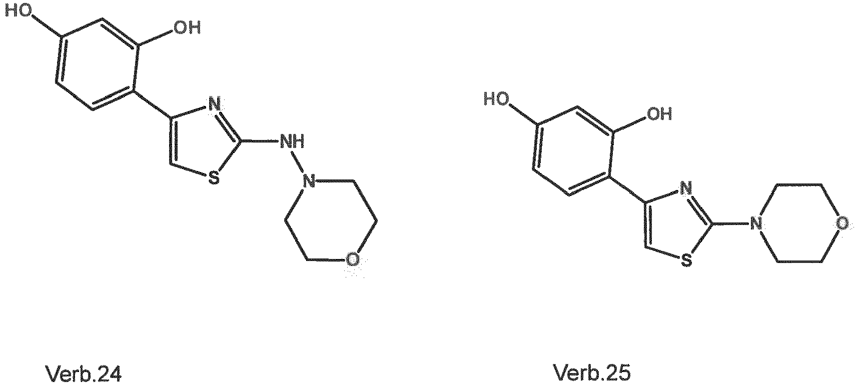

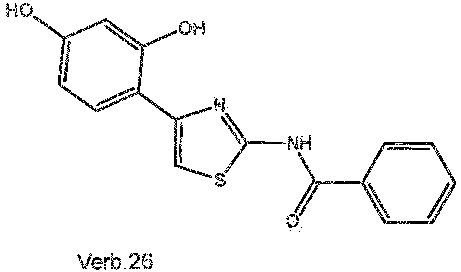

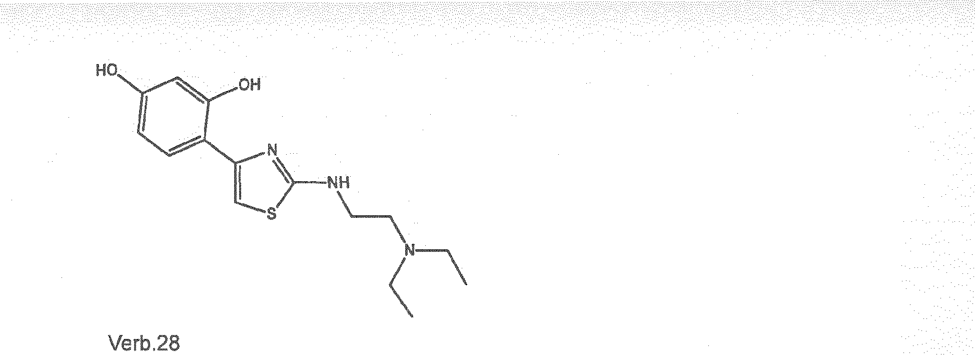

2. Zubereitungen nach Anspruch 1, **dadurch gekennzeichnet, dass** das oder die Thiazole als Halogenid, ein Carbonat, ein Ascorbat, ein Acetat oder ein Phosphat vorliegt oder vorliegen.

3. Zubereitungen nach einem der vorstehenden Ansprüche, **dadurch gekennzeichnet, dass** sie 0,00001 Gew.-% bis 10 Gew.-%, bevorzugt 0,001 Gew.-% bis 3,0 Gew.-%, insbesondere 0,005 - 1,0 Gew.-%, bezogen auf das Gesamtgewicht der Zubereitungen, an einem oder mehreren Thiazolen enthalten.

4. Thiazole, oder Zubereitungen, eines oder mehrere solcher Thiazole gemäß Anspruch 1 enthaltend, zur Verwendung bei der Behandlung und/oder Prophylaxe unerwünschter Hautpigmentierung.

## Claims

1. Cosmetic or dermatological preparations having an active content of one or more thiazoles which is or are selected from the group of the following substances:

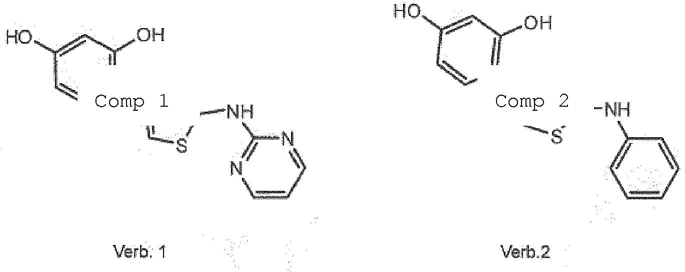

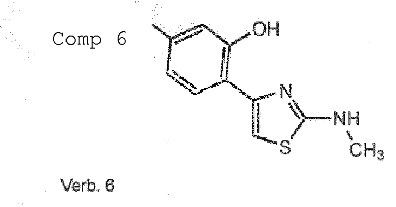

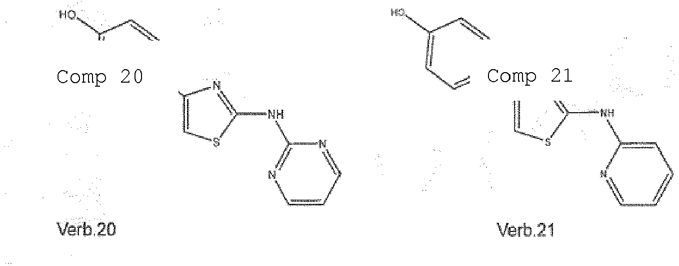

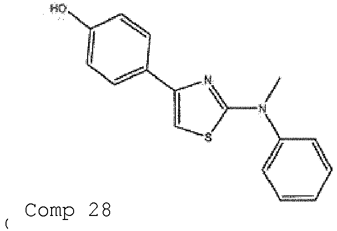

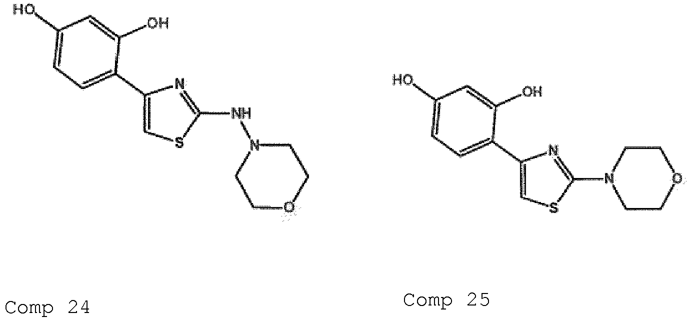

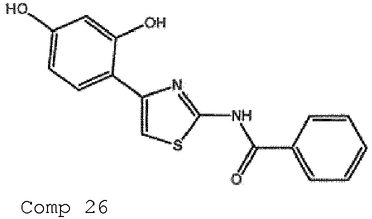

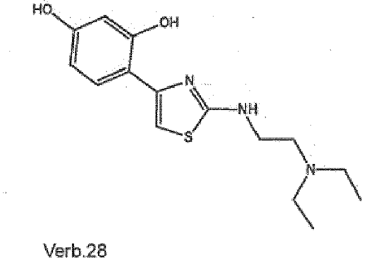

2. Preparations according to Claim 1, **characterized in that** the thiazole(s) is or are present as halide, a carbonate, an ascorbate, an acetate or a phosphate.

3. Preparations according to either of the preceding claims, **characterized in that** said preparations comprise 0.00001 % by weight to 10% by weight, preferably 0.001% by weight to 3.0% by weight, especially 0.005 - 1.0% by weight of one or more thiazoles, based on the total weight of the preparations.

4. Thiazoles or preparations comprising one or more such thiazoles according to Claim 1 for use in the treatment and/or prophylaxis of undesired skin pigmentation.

## Revendications

1. Préparations cosmétiques ou dermatologiques ayant une teneur efficace en un ou plusieurs thiazoles qui sont choisis dans le groupe constitué par les substances suivantes :

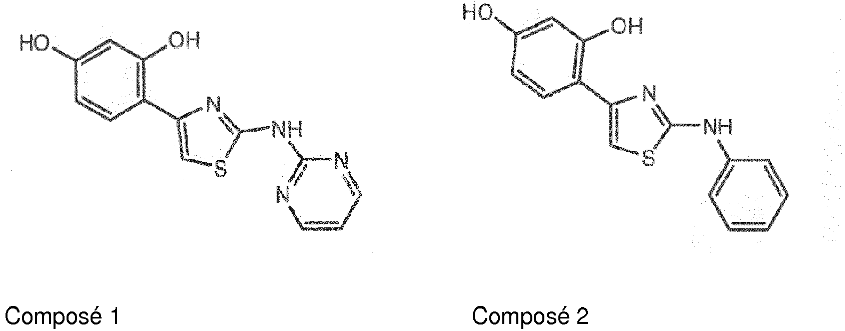

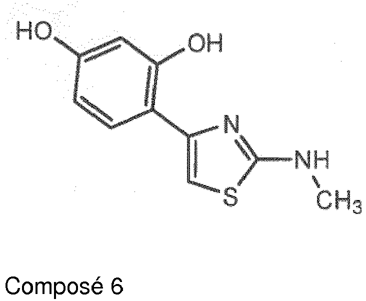

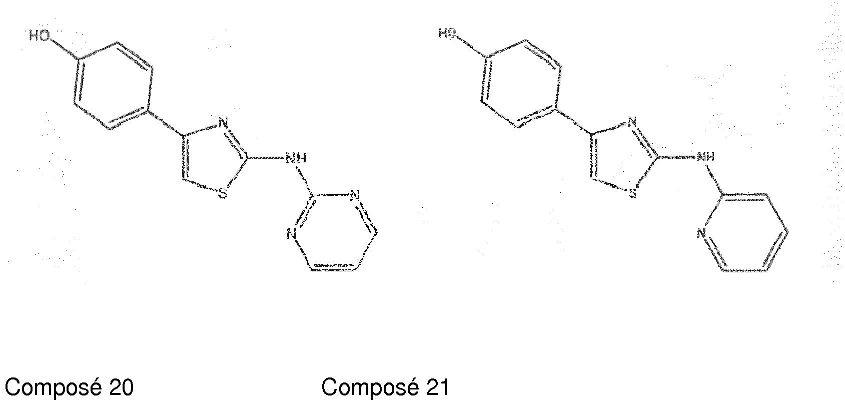

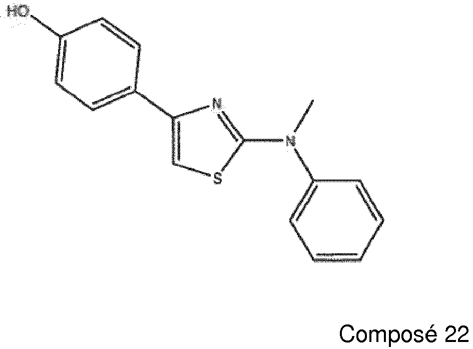

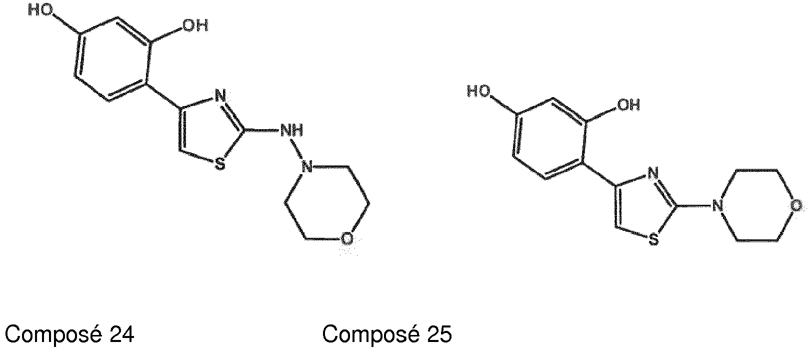

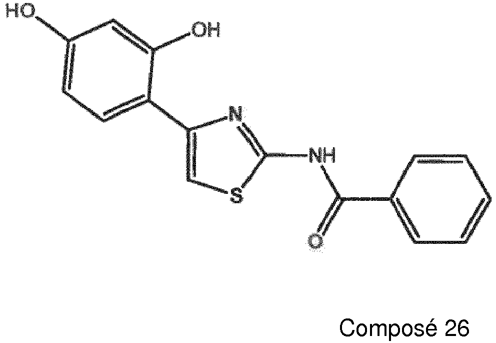

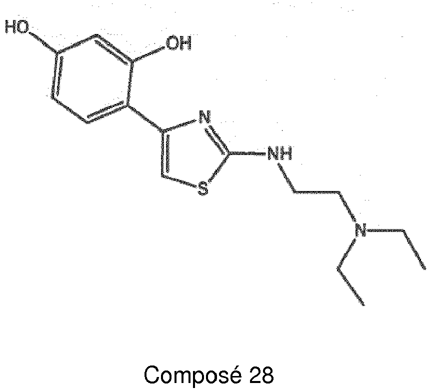

2. Préparations selon la revendication 1, **caractérisées en ce que** le ou les thiazoles se présentent sous la forme d'un halogénure, d'un carbonate, d'un ascorbate, d'un acétate ou d'un phosphate.

3. Préparations selon l'une quelconque des revendications précédentes, **caractérisées en ce qu'**elles contiennent 0,00001 % en poids à 10 % en poids, de préférence 0,001 % en poids à 3,0 % en poids, notamment 0,005 à 1,0 % en poids, par rapport au poids total des préparations, d'un ou de plusieurs thiazoles.

4. Thiazoles, ou préparations contenant un ou plusieurs tels thiazoles selon la revendication 1, pour une utilisation lors du traitement et/ou de la prophylaxie d'une pigmentation indésirable de la peau.
